# EUROPEAN PATENT SPECIFICATION

(11) **EP 3 031 401 B1**
(45) Date of publication and mention of the grant of the patent: **05.07.2017**
(21) Application number: 15197825.1
(22) Date of filing: 03.12.2015
(51) Int. Cl.: A61B 17/02, A61B 1/06

(54) **ILLUMINATING MEDICAL INSTRUMENT**
BELEUCHTENDES MEDIZINISCHES INSTRUMENT
INSTRUMENT MÉDICAL D'ÉCLAIRAGE

(30) Priority: 12.12.2014 IT MI20142130
(43) Date of publication of application: 15.06.2016
(73) Proprietor: Piciche', Marco, 00199 Roma (IT)
(72) Inventor: Piciche', Marco, 00199 Roma (IT)
(74) Representative: Mittler, Andrea

(56) References cited:
- US-A1- 2005 063 177
- US-A1- 2014 221 754

## Description

The present invention relates to an illuminating medical instrument.

Having adequate lighting is necessary for surgical operations. In the known art, the lamps of an operating room have the disadvantage of needing to be continuously adjusted to be oriented towards a specific area to be illuminated and the surgical operation must be continuously interrupted to adjust said lamps. The lamp may make dust or dirt fall into the area to be illuminated, thus disadvantageously creating problems of sterility. Especially for surgical operations in deep areas of a body, obtaining good lighting is difficult and surgeons often fit a lamp on their heads. However, such lamps on the surgeons' heads are poorly tolerated by many surgeons who complain of headaches.

US-2014/0221754 describes a flexible light which can be mounted directly on a medical instrument provided with electric connections of the female type. The flexible lamp may orient the light and hold the orientation of the same over time, however it disadvantageously creates problems from the point of view of the sterile cleanliness needed for surgical operations; indeed, the female connection collects dirt and is difficult to clean in sterile manner. The lamp must be disadvantageously oriented each time to the extent of having to interrupt the surgical operation continuously in order to adjust the lamp.

US-6585727-B1 describes a light wave guide mounted directly over the body of a medical instrument so as to be able to illuminate in a given preset direction. Disadvantageously, said wave guide is not very flexible because said wave guide cannot be oriented in other directions with respect to the one preset by the medical instrument because such a wave guide follows the orientation of the medical instrument. During the surgical operation, other lights must be prepared for illuminating other surgical areas on which to operate. Furthermore, said wave guide mounted externally on the body of the medical instrument creates problems of sterile cleanliness. The sterile cleanliness of the medical instrument is necessary to avoid the passage of microbes and bacteria from one patient to another in order to avoid dangerous complications.

EP-2246000-B1 describes a light wave guide or, respectively, a supply cable for a light-emitting diode (LED) mounted inside a body of a medical instrument but disadvantageously the light cannot be oriented in a different direction from that of a second arm of the medical instrument which illuminates a first arm thereof. Disadvantageously, there are problems of sterile cleaning of the medical instrument at the outlet of the light from the wave guide or at the LED, respectively.

EP-0993579-B1 describes a light wave guide for a medical instrument, said light wave guide is mounted within the instrument and follows the shape of the instrument, orienting a maximum intensity of the light beam in a predetermined direction dictated by the shape of the medical instrument. A multiplicity of light diffusers is provided at a light portion on a reflecting surface of the wave guide which allow to diffuse part of the light beam into a multiplicity of directions in addition to that fixed by the shape of the instrument. Said part of the light beam has a lesser light intensity than the predetermined direction in which there is the maximum light beam intensity. Disadvantageously, the orientation of the light beam cannot be modified as desired and the maximum intensity of the light beam cannot be oriented towards other directions as desired.

EP-0993579-B1 and US-6739744-B2 describe a flexible light adapted to modify the orientation of the maximum intensity of the light beam as desired but has the disadvantage of having to interrupt the surgical operation to change the direction of said flexible light. Furthermore, said flexible light has a male connector which is mounted on a female connector, which has the disadvantage of being a receptacle for dirt, microbes and bacteria, to the extent of being disadvantageously difficult to clean in sterile manner.

It is the object of the present invention to make a medical instrument comprising an illuminator which can be cleaned in sterile manner, capable of orienting a light beam into a multiplicity of directions with respect to the medical instrument as desired without needing to interrupt a surgical operation, capable of illuminating in depth an area of a body subjected to a surgical operation, and capable of adjusting the light intensity of the light beam.

It is a further object of the present invention to make a sternal mammary retractor comprising an illuminator which can be cleaned in sterile manner, capable of orienting a light beam in a multiplicity of directions as desired with respect to an arm of the sternal mammary retractor without needing to interrupt a surgical operation, capable of illuminating in depth a thoracic hemisternum and a bed of an internal thoracic artery to be taken located in depth, and capable of adjusting the light intensity of the light beam.

According to the invention, such objects are reached by a medical instrument according to claim 1.

These and other features of the present invention will become more apparent from the following detailed description of a practical embodiment thereof, shown by way of non-limitative example in the accompanying drawings, in which:
Figure 1 shows a top plan view of a medical instrument, e.g. a sternal retractor,
Figure 2 shows a top plan view of a medical instrument according to a first alternative,
Figure 3 shows a top plan view of a medical instrument according to a second alternative,
Figure 4 shows a section view taken along lines IV-IV of Figure 1,
Figure 5 shows a section view taken along lines V-V of Figure 1,
Figure 6 shows a section view taken along lines VI-VI of Figure 1,
Figure 7 shows a side plan view of a wall on which light sources of the medical instrument are mounted,
Figure 8 shows a side plan view of an alternative wall on which light sources disposed in alternating manner of the medical instrument are mounted,
Figure 9 shows a magnified detail A of figure 1,
Figure 10 shows a section view taken along lines X-X of Figure 9.

The figures listed above show a medical instrument, in particular a sternal mammary retractor 1, comprising two arms 10-12 to open a portion of the human body and surgically operate in a chest in depth.

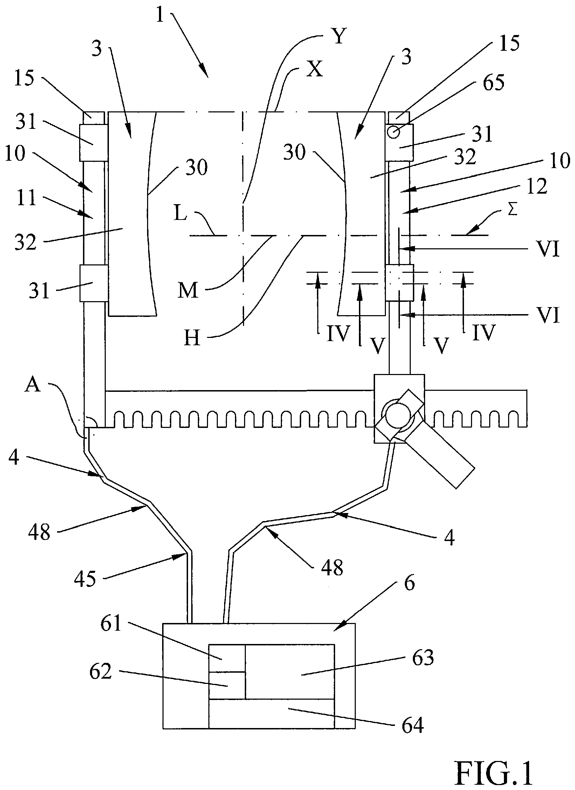

As shown in particular in Figures 1-3, which illustrate different medical sternal retractor instruments, it may be observed that the two arms 10-12 are a first arm 11 and a second arm 12, which are suitable for passing from a position of minimum mutual distance to a position of maximum mutual distance between each other 11, 12. Said a mutual distance is measured along a horizontal axis X, as shown in Figure 1.

Each arm 10 of said two arms 10-12 extends longitudinally along a direction of a longitudinal axis Y. Wherein said longitudinal axis Y is perpendicular with respect to said horizontal axis X. Each arm 10 comprising a longitudinal axis 15 which extends in direction of longitudinal axis Y.

The arm 10-12 comprises at least one prehensile portion 3 mounted in sliding manner with said longitudinal body 15 of the arm 10-12.

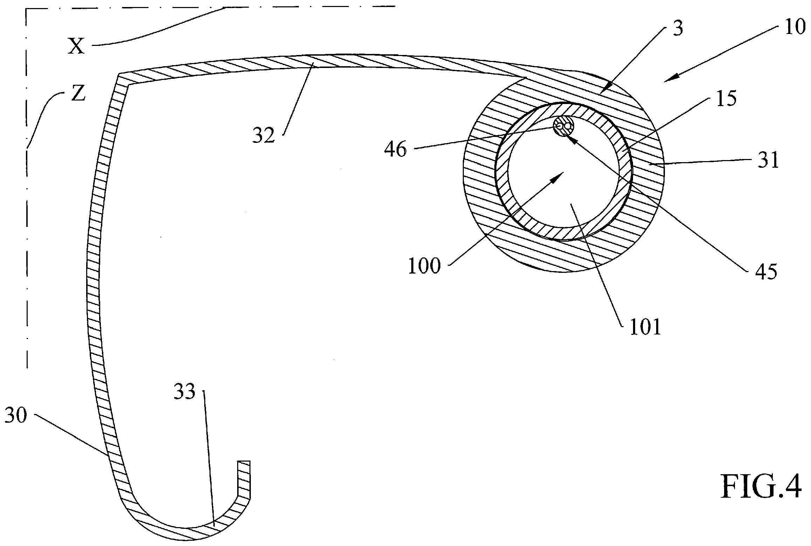

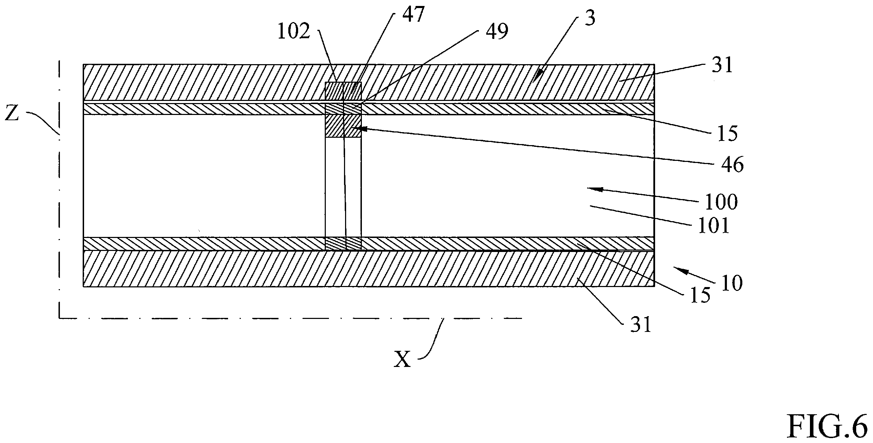

As shown in particular in Figures 4-6, the longitudinal body 15 has a cylindrical section comprising a first portion of through cavity 101 carved along the longitudinal axis Y. At least one cavity 100 of the arm 10-12 comprises said first portion of cavity 101 carved inside said longitudinal body 15 along the longitudinal axis Y of said at least one arm 10.

As shown in particular in Figures 4-5, the prehensile portion 3 comprises a first cylindrical portion 31 slidingly mounted above said longitudinal body 15 so as to be able to make the prehensile device turn about the longitudinal axis Y. The prehensile portion 3 also comprises a second horizontal portion 32 integrally connected to the first cylindrical portion 31. Said horizontal portion 32 is connected at the end thereof with a vertical wall 30 pending downwardly along a direction of a vertical axis Z, where said vertical axis Z is perpendicular both to the horizontal axis X and to the longitudinal axis Y. Said vertical axis Z goes deep into the chest or open portion of the body on which to surgically operate. The vertical wall 30 is connected by a lower end thereof to a hook 33 for coupling better with an end of the body and holding it in position during the surgical operation.

The cavity 100 also comprises a second portion of the cavity 102 carved inside said wall 30, of said horizontal portion 32 and of said cylindrical portion 31 of the prehensile portion 3 of said at least one arm 10.

Each arm 10-12 comprises at least one of said prehensile portions 3, comprising at least one of said wall 30 pending downwards.

At least one arm 10 of said at least two arms 10-12 mounts a lighting apparatus 4.

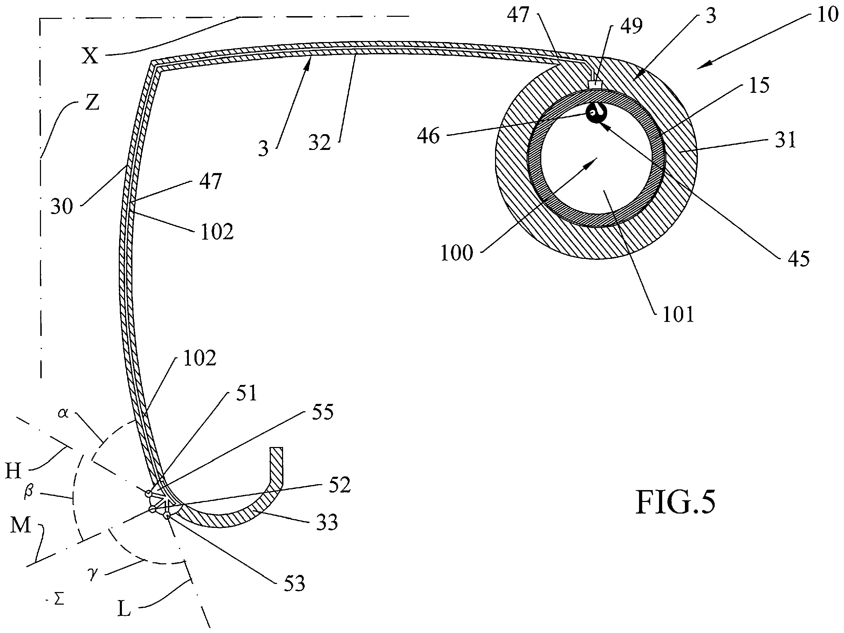

As shown in particular in Figures 5, 7-8, said lighting apparatus 4 comprises a multiplicity of lighting sources 51-53 disposed along said wall 30.

The lighting sources 51-53 are disposed so that at least one first lighting source 51 of said multiplicity of lighting sources 51-53 is disposed at a height which is more elevated with respect to at least one second lighting source 52 of said multiplicity of lighting sources 51-53 and at least one third lighting source 53 of said multiplicity of lighting sources 51-53 is disposed at a height which is lower with respect to said at least one second lighting source 52. Said height is measurable along the vertical axis Z.

As shown in particular in Figure 5, said multiplicity of lighting sources 51-53 emit a multiplicity of light beams, whose maximum intensities are directed along a multiplicity of mutually diverging axes H, M, L disposed on a geometrical plane Σ identified by the horizontal axis X and by the vertical axis Z.

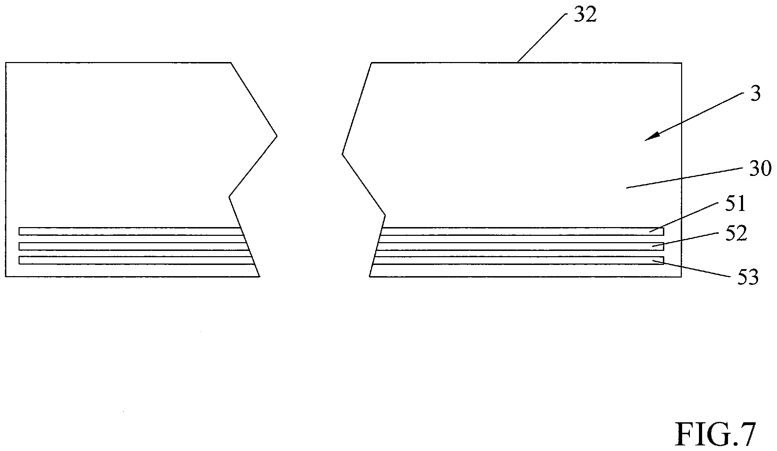

As shown in particular in Figure 7, said first 51, second 52 and third 53 lighting sources are respectively a longitudinal light, which may be neon or other gas light, or a series of light-emitting diodes (LED) disposed in a row along longitudinal axis Y. The first light source 51 is disposed at a greater height with respect to the second lighting source 52, which is disposed at a height which is more elevated with respect to the light source 53.

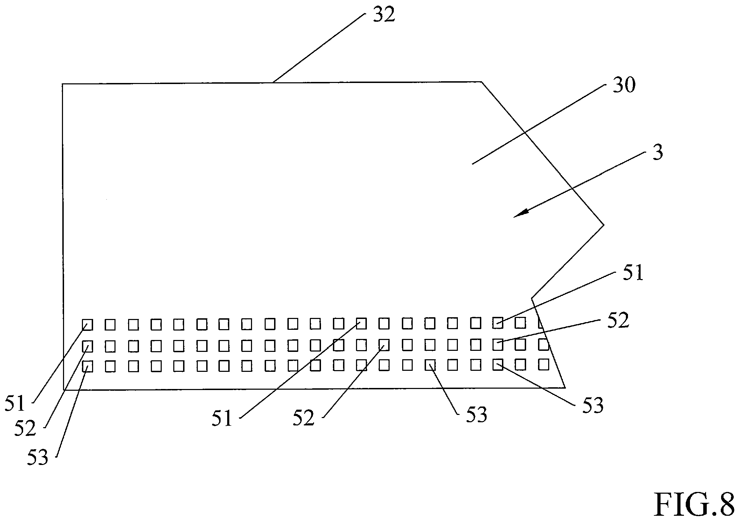

As shown in particular in Figure 8, the first 51, second 52 and third lighting sources 53 could be a multiplicity of lighting sources disposed on longitudinal rows along the longitudinal axis Y, so that a first longitudinal row of a multiplicity of first lighting sources 51 is at a height which is more elevated with respect to a second longitudinal row of second lighting sources 52, which, in turn, is disposed at a height which is more elevated with respect to a third longitudinal row of third lighting sources 53.

Said lighting sources 51-53 may be neon or other gas lights, LEDs or light bulbs or light wave guides.

As shown in particular in Figure 5, said at least one first lighting source 51 emits a first light beam, whose maximum intensity is directed along a first diverging axis H and said at least one second lighting source 52 emits a second light beam, whose maximum intensity is directed along a second diverging axis M, said at least one third lighting source 53 emits a third light beam whose maximum intensity is directed along a third axis L. Said first axis H, said second axis M and said third axis L lie on the geometric plane Σ.

As shown in particular in Figure 5, said first diverging axis H is directed at a first angle α with respect to said wall 30. Said first angle α lies on said geometrical plane Σ and is comprised between 0 and 90 sexagesimal degrees.

Said second diverging axis M is directed at a second angle β with respect to said first axis H, said second angle β lies on said geometrical plane Σ and is comprised between 0 and 270 sexagesimal degrees.

Said third diverging axis L is directed at a third angle β with respect to said second axis M, said third angle γ lies on said geometrical plane Σ and is comprised between 0 and 270 sexagesimal degrees.

There may be more than three rows of lighting sources 51-53 so as to illuminate different deep portions of the part of the human body on which to surgically operate.

Advantageously, said multiplicity of lighting sources 51-53 emit a multiplicity of lighting beams whose maximum intensities are directed along a mutually diverging multiplicity of axes H, M, L to better illuminate different depths of the opening of the body subjected to a surgical operation.

Said lighting apparatus 4 is integrally mounted with said at least one arm 10 of the medical instrument 1. The lighting apparatus 4 follows a shape of said at least one arm 10.

Advantageously, the lighting apparatus 4 mounted integrally with the arm 10 does not need to be repositioned by hand in order to be oriented each time along a different direction to illuminate portions of the body at different depth.

Advantageously, the fact that the lighting apparatus 4 is mounted integrally with the arm 10 allows not to move said arm 10 and not to move the wall 30 needed to keep an edge of the body subjected to a surgical operation stationary.

The lighting apparatus 4 comprises at least one power supply cable 45 mountable inside at least one cavity 100 of said at least one arm 10 of said medical instrument 1. Said multiplicity of lighting sources 51-53 are electrically connected to said at least one power supply cable 45. Advantageously, said at least one electrical power supply cable 45 is mounted within the cavity 100 so as to protect it from the highly corrosive environment of the surgical operation. Furthermore, disposing at least one electrical supply cable 45 within the arm 10-12 allows to clean the entire medical instrument 1 in sterile manner very advantageously and simply, e.g. with an autoclave.

An external mounting of the lighting apparatus 4 on the arm 10-12 does not allow to obtain cleanliness on sterile level suited to use the medical instrument 1 effectively for surgical operations on different individuals, because there would be the risk of passing microbes or bacteria from one individual to another, thus creating a dangerous health risk.

Said at least one power supply cable 45 comprises at least one inner portion of a power supply cable 46, 47 mounted inside said at least one cavity 100 of said at least one arm 10 in which the lighting apparatus 4 is mounted.

Said at least one power supply cable 45 also comprises an outer portion of electric supply cable 48 connected to an external power supply.

Said outer portion of a power supply cable 48 is covered with cellophane which can be removed to allow to obtain the cleanliness level at surgical sterility levels.

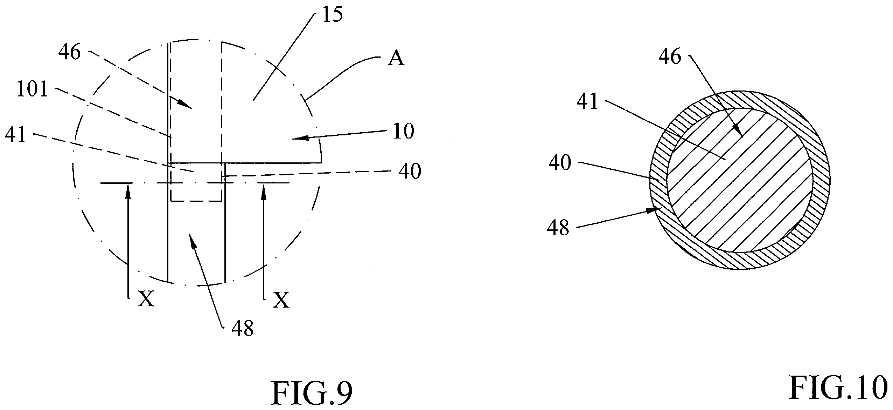

As shown in particular in Figures 9-10, said outer portion of the power supply cable 48 comprises an extremity with female plug 40.

Said at least one inner portion of the power supply cable 46, 47 comprises an extremity with male plug 41. Said extremity with male plug 41 extends out of said at least one arm 10 to be connected to said female plug 40 of said outer portion of power supply cable 48. Advantageously, the male connection 41 allows to obtain facilitated cleanliness on sterile level, because no dirt, bacteria or microbes can settle in the male connector 41, unlike in a female connector. Advantageously, the male connector 41 is an outer extension of the medical instrument 1 to facilitate cleaning operations on sterile cleanliness level in advantageous manner, because an autoclave may be used.

As shown in particular in Figures 4-6, 9-10, said at least one electrical power supply cable 45 comprises a first inner portion of electrical power supply cable 46 mounted inside the first cavity portion 101.

Said at least one electrical power supply cable 45 also comprises a second inner portion of the power supply cable 47 mounted inside the second portion of cavity 102 and a brush 49 electrical connector for electrically connecting the first portion 46 and the second portion of the power supply cable 47 to transmit the electric supply current to the multiplicity of lighting sources 51-53 connected to the ends of the second portion of electrical power supply cable 47.

At the end of the second electrical power supply portion 47, said multiplicity of lighting sources 51-53 are mounted inside a sterile seal 55 to allow sterile level cleaning of the medical instrument 1 in advantageous manner. Said sterile seal 55 does not have slits which could compromise the cleanliness of the medical instrument 1 such to allow the introduction of bacteria and microbes which are difficult to clean in sterile manner. Said sterile seal 55 is made of a material which is resistant to the highly corrosive environment of the surgical operation.

As shown in Figures 1-3, said multiplicity of lighting sources 51-53 are logically connected to an electronic instrument 6, e.g. a computer 6, comprising at least one storage 61 and at least one processor 62.

The computer 6 may also include an interface 63, 64 for entering data in storage 61, said interface 63, 64 comprising a screen 63 and a keyboard 64.

Said at least one storage 61 contains steps of at least one first procedure executed by said at least one processor 62.

Said at least one first procedure comprising at least one first switch-on step of said at least one first lighting source 51 of said multiplicity of lighting sources 51-53 for at least one first period of time T1 and at least one second switch-on step of said at least one second lighting source 52 of said multiplicity of lighting sources 51-53 for at least one second period of time T2, said periods of time T1, T2 being set in said at least one storage 61, by means of the interface 63, 64 or by means of a remote control 65 mounted on the medical instrument 1 and easily reachable by the surgeon or by another operator.

At least one third switch-on step of said at least one third lighting source 53 of said multiplicity of lighting sources 51-53 may be provided for at least one third period of time T3.

The setting may be modified during the operation directly by the surgeon by means of the remote control 65 or by an operator controlling the processor 62 by means of the interface 63, 64. The storage 61 may be set also before the surgical operation for automating the switch-on and switch-off procedure of the lighting sources 51-53 for different periods of time T1,T2.

Said at least one processor 62 implements at least said first switch-on step and at least said at least one second switch-on step sequentially or simultaneously, so that the lights 51-53 are all on or it is possible to switch on only one row of lights 51-53 to illuminate only a deep portion of the body during the surgical operation.

It is advantageously possible to adjust also the intensity of each of said rows of lights 51-53; indeed, said at least one storage 61 comprises steps of a second procedure executed by said at least one processor 62, said second procedure comprising at least one first step for increasing a lighting intensity of at least one lighting source 51-53 of said multiplicity of lighting sources 51-53 and at least a second step for decreasing the lighting intensity of said at least one lighting source 51-53.

The remote control device 65 commands said processor 62, sets said periods of time T1, T2, T3 into said at least one storage 61, sets into said at least one storage 61 a sequence of steps to switch on or switch off any of said multiplicity of light sources 51-53 and steps for adjusting the lighting intensity of any of said multiplicity of lighting sources 51-53. The operations of the remote control device 65 may also be performed by an operator using the control interface 63, 64.

For the operation of the medical instrument 1, it is possible to set the storage 61 so that the processor 62 switches on any lighting source 51-53 sequentially or simultaneously so as to illuminate the entire depth of the body subject to surgical operation or a single portion at different depth. The storage 61 may be set and the process 62 may be controlled either before the surgical operation or during the surgical operation.

Advantageously, the storage 61 and the processor 62 allow to automate the lighting of the deep portions of the body subject to surgical operation without needing to interrupt the surgical operation and without continuing to manually orient the lighting sources 51-53 which instead remain fixed and pre-oriented in mutually diverging manner to facilitate the lighting of the deep portions of the body.

Alternatively, the wall 30 must be mounted in sliding manner directly with the longitudinal body 15 of the arm 10 and said wall 30 pending downwards so as to be able to engage an edge of the portion of the body on which the surgical operation is performed.

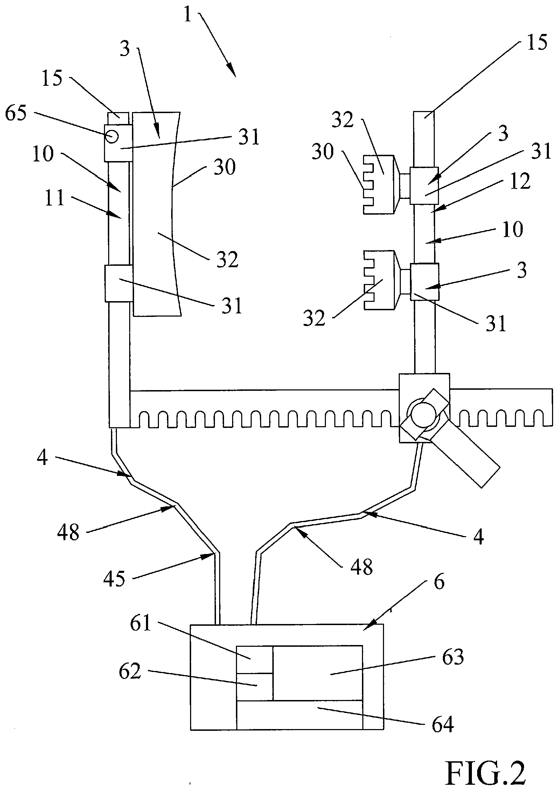

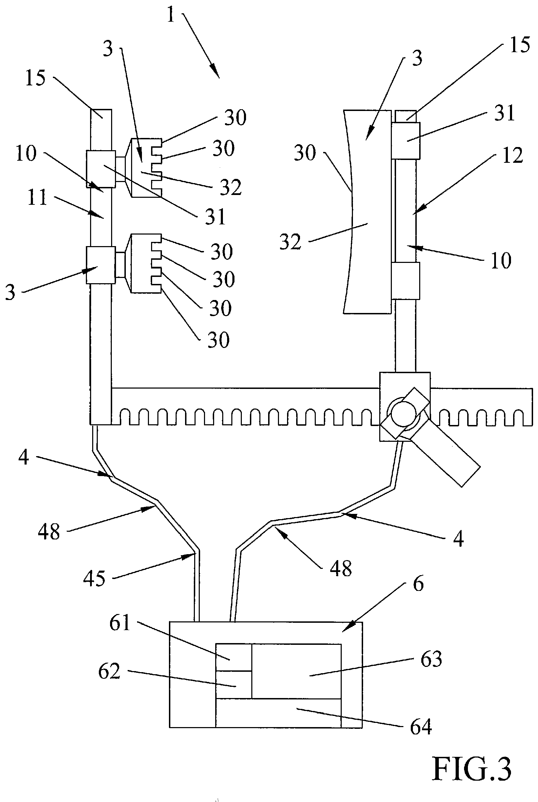

According to a further alternative, said medical instrument 1 is chosen from a list which comprises a retractor 1, a sternal retractor 1, a sternal mammary retractor 1, a pair of scissors 1 or a pair of tweezers 1, as shown in Figures 2 and 3, for example. In said further alternative, there are two or more prehensile devices 3, thus there are also two or more walls 30. The prehensile devices 3 may also fit three or more walls 30, as shown in Figures 2-3.

In a yet further alternative, there may be more than two arms 10-12, in particular operations in which at least one third arm is needed (not shown in the figures), which may be disposed, according to needs and the size of the opening in the body, also along the horizontal axis X to better retract a part of the body subject to a surgical operation.

In a yet further alternative, the lighting apparatus 4 can be mounted on all the arms 10-12 and on all the walls 30.

In a yet further alternative, the lighting apparatus 4 may be mounted also outside the arm 10-12 following the profile of the shape of the arm 10-12. In said case, in order to maintain cleanliness on sterile level of the medical instrument 1, the lighting apparatus must be covered with sterile material, e.g. by entirely covering it with cellophane so that the cellphone may be removed and checked or cleaned in sterile manner.

Advantageously, the medical instrument 1 according to the present invention can be cleaned in sterile manner throughout, can orient a lighting beam in a multiplicity of directions as desired with respect to the medical instrument 1 without needing to interrupt a surgical operation, can illuminate in depth an area of the body subjected to surgery, and can adjust the intensity of the light source of each of the light beams of each of said multiplicity of lighting sources 51-53 so as to facilitate the surgical operation.

Advantageously, when the medical instrument 1 is a sternal mammary retractor, it can be cleaned in sterile manner, can orient the lighting beams of a multiplicity of lighting sources 51-53 in a multiplicity of diverging directions H, L, M as desired with respect to an arm 10 of the sternal mammary retractor 1 without needing to interrupt the surgical operation, can illuminate in depth a thoracic hemisternum subjected to a surgical operation to illuminate fully and in depth the mammary thoracic artery vessels located deep in the thoracic hemisternum, otherwise invisible with the instruments present in the known art, and can adjust the light intensity of each of the lighting beams of each of said multiplicity of lighting sources 51-53 so as to be able to better see portions of body at different depths which would otherwise not be illuminated efficiently for the surgical operation and to see the small vessels of the thoracic artery that constant lighting would not allow to see or highlight with respect to the rest of the body.

Advantageously, the medical instrument 1 may be used for coronary surgery (with beating heart or not), because in beating heart surgery the heart is dislocated in given positions and therefore some coronaries are difficult to access visually; furthermore, that medical instrument 1 may be advantageously used for very deep mitral valve and for aortic valve surgery.

## Claims

1. Medical instrument (1) comprising at least two arms (10-12), at least one first arm (11) and at least one second arm (12) which are suitable for passing from a position of minimum mutual distance to a position of maximum mutual distance between each other (11, 12), said mutual distance being measured along a horizontal axis (X), each arm (10) of said at least two arms (10-12) extends longitudinally along a direction of a longitudinal axis (Y) being perpendicular with respect to said horizontal axis (X), each arm (10-12) comprises at least one prehensile portion (3) comprising a wall (30) pending downwardly along a direction of a vertical axis (Z), said vertical axis (Z) is perpendicular both to the horizontal axis (X) and to the longitudinal axis (Y), at least one arm (10) of said at least two arms (10-12) mounts a lighting apparatus (4) which comprises a multiplicity of lighting sources (51-53) disposed along said wall (30), **characterized in that** at least one first lighting source (51) of said multiplicity of lighting sources (51-53) is disposed at a height which is more elevated with respect to at least one second lighting source (52) of said multiplicity of lighting sources (51-53), said height is measurable along the vertical axis (Z), said multiplicity of lighting sources (51-53) emit a multiplicity of light beams whose maximum intensities are directed along a multiplicity of axes (H, M, L) diverging each other and disposed on a geometrical plane (Σ) identified by the horizontal axis (X) and by the vertical axis (Z).

2. Medical instrument (1) according to claim 1, **characterized in that** said at least one first lighting source (51) emits a first light beam whose maximum intensity is directed along a first diverging axis (H) of said multiplicity of axes (H, M, L) and said at least one second lighting source (52) emits a second light beam whose maximum intensity is directed along a second diverging axis (M) of said multiplicity of axes (H, M, L), said first diverging axis (H) is directed at a first angle (α) with respect to said wall (30), said first angle (α) lying on said geometrical plane (Σ) and being comprised between 0 and 90 sexagesimal degrees and said second diverging axis (M) is directed at a second angle (β) with respect to said first axis (H), said second angle (β) lying on said geometrical plane (Σ) and being comprised between 0 and 270 sexagesimal degrees.

3. Medical instrument (1) according to any one of the preceding claims 1 or 2, **characterized in that** said lighting apparatus (4) comprises at least one power supply cable (45) mountable inside of at least one cavity (100) of said at least one arm (10) of said medical instrument (1), said multiplicity of lighting sources (51-53) are electrically connected to said at least one power supply cable (45).

4. Medical instrument (1) according to claim 3, **characterized in that** said power supply cable (45) comprises at least one inner portion of a power supply cable (46, 47) mounted inside of said at least one cavity (100) of said at least one arm (10) and an outer portion of a power supply cable (48) connected to an external power supply, said outer portion of the power supply cable (48) comprises an extremity with female plug (40) and said at least one inner portion of the power supply cable (46, 47) comprises an extremity with male plug (41), said extremity with male plug (41) extends out of said at least one arm (10) to be connected with said female plug (40) of said outer portion of power supply cable (48).

5. Medical instrument (1) according to any one of the preceding claims 3-4, **characterized in that** said at least cavity (100) comprises a first portion of cavity (101) carved inside a longitudinal body (15) of said at least one arm (10) and a second portion of the cavity (102) carved inside said wall (30) of said at least one arm (10), said wall (30) slidably mounted with said longitudinal body (15), said at least one power supply cable (45) comprising a first inner portion of the power supply cable (46) mounted inside the first portion of cavity (101), a second inner portion of the power supply cable (47) mounted inside the second portion of cavity (102) and a brush (49) electrical connector for electrically connecting the first portion (46) and the second portion of the power supply cable (47).

6. Medical instrument (1) according to any one of the preceding claims 1-5, **characterized in that** said multiplicity of lighting sources (51-53) are mounted inside a steril seal (55).

7. Medical instrument (1) according to any one of the preceding claims, **characterized in that** said multiplicity of lighting sources (51-53) are logically connected with an electronic instrument (6) comprising at least one storage (61) and at least one processor (62), said at least one storage (61) comprises steps of at least one first procedure executed by said at least one processor (62), said at least one first procedure comprising at least one first ignition step of said at least one first lighting source (51) of said multiplicity of lighting sources (51-53) for at least one first period of time (T1) and at least one second ignition step of said at least one second lighting source (52) of said multiplicity of lighting sources (51-53) for at least one second period of time (T2), said periods of time (T1, T2) being set into said at least one storage (61).

8. Medical instrument (1) according to claim 7, **characterized in that** said at least one processor (62) executes at least said at least one first ignition step and at least one said at least one second ignition step sequentially or simultaneously.

9. Medical instrument (1) according to any one of the preceding claims 7 or 8, **characterized in that** said at least one storage (61) comprises steps of a second procedure executed by said at least one processor (62), said second procedure comprising at least one first step for increasing a lighting intensity of at least one lighting source (51-53) di said multiplicity of lighting sources (51-53) and at least a second step for decreasing the lighting intensity of said at least one lighting source (51-53).

10. Medical instrument (1) according to any one of the preceding claims 7-9, **characterized in that** a remote control device (65) commands said processor (62), sets into said at least one storage (61) said periods of time (T1, T2), sets into said at least one storage (61) a sequence of steps.

## Patentansprüche

1. Medizinisches Instrument (1), aufweisend wenigstens zwei Arme (10-12), wenigstens einen ersten Arm (11) und wenigstens einen zweiten Arm (12), die für einen Übergang von einer Stellung eines minimalen gegenseitigen Abstandes in eine Stellung eines maximalen gegenseitigen Abstandes zueinander (11, 12) geeignet sind, wobei der gegenseitige Abstand entlang einer horizontalen Achse (X) gemessen ist, wobei jeder Arm (10) der wenigstens zwei Arme (10-12) sich longitudinal entlang einer Richtung einer longitudinalen Achse (Y) erstreckt, die orthogonal bezüglich der horizontalen Achse (X) ist, wobei jeder Arm (10-12) wenigstens einen zum Greifen geeigneten Abschnitt (3) aufweisend eine nach unten entlang einer Richtung einer vertikalen Achse (Z) hängende Wand (30) aufweist, wobei die vertikale Achse (Z) orthogonal sowohl bezüglich der horizontalen Achse (X) als auch zu der longitudinalen Achse (Y) ist, wobei wenigstens ein Arm (10) der wenigstens zwei Arme (10-12) eine Beleuchtungsvorrichtung (4) lagert, die eine Mehrzahl von entlang der Wand (30) angeordneten Beleuchtungsquellen (51-53) aufweist, **dadurch gekennzeichnet, dass** wenigstens eine erste Beleuchtungsquelle (51) der Mehrzahl von Beleuchtungsquellen (51-53) in einer Höhe angeordnet ist, die bezüglich wenigstens einer zweiten Beleuchtungsquelle (52) der Mehrzahl von Beleuchtungsquellen (51-53) weiter oben liegt, wobei die Höhe entlang der vertikalen Achse (Z) messbar ist, wobei die Mehrzahl von Beleuchtungsquellen (51-53) eine Mehrzahl von Lichtbündeln emittiert, deren maximale Intensitäten entlang einer Mehrzahl von Achsen (H, M, L) gerichtet sind, die zueinander divergieren und auf einer durch die horizontale Achse (X) und durch die vertikale Achse (Z) bestimmten geometrischen Ebene (Σ) angeordnet sind.

2. Medizinisches Instrument (1) nach Anspruch 1, **dadurch gekennzeichnet, dass** die wenigstens eine erste Beleuchtungsquelle (51) ein erstes Lichtbündel emittiert, dessen maximale Intensität entlang einer ersten divergierenden Achse (H) der Mehrzahl von Achsen (H, M, L) gerichtet ist und die wenigstens eine zweite Beleuchtungsquelle (52) ein zweites Lichtbündel emittiert, dessen maximale Intensität entlang einer zweiten divergierenden Achse (M) der Mehrzahl von Achsen (H, M, L) gerichtet ist, wobei die erste divergierende Achse (H) in einem ersten Winkel (α) bezüglich der Wand (30) gerichtet ist, wobei der erste Winkel (α) auf der geometrischen Ebene (Σ) liegt und zwischen 0° und 90° liegt, und wobei die zweite divergierende Achse (M) in einem zweiten Winkel (β) bezüglich der ersten Achse (H) gerichtet ist, wobei der zweite Winkel (β) auf der geometrischen Ebene (Σ) liegt und zwischen 0° und 270° liegt.

3. Medizinisches Instrument (1) nach einem der vorangehenden Ansprüche 1 oder 2, **dadurch gekennzeichnet, dass** die Beleuchtungsvorrichtung (4) wenigstens ein Stromversorgungskabel (45) aufweist, das im Inneren wenigstens einer Kavität (100) des wenigstens einen Arms (10) des medizinischen Instruments (1) einbaubar ist, wobei die mehreren Beleuchtungsquellen (51-53) mit dem wenigstens einen Stromversorgungskabel (45) elektrisch verbunden sind.

4. Medizinisches Instrument (1) nach Anspruch 3, **dadurch gekennzeichnet, dass** das Stromversorgungskabel (45) wenigstens einen Innenabschnitt einer Stromversorgungsleitung (46, 47), der im Inneren der wenigstens einen Kavität (100) des wenigstens einen Arms (10) eingebaut ist, und einen Aussenabschnitt einer Stromversorgungsleitung (48) aufweist, der mit einer externen Stromversorgung verbunden ist, wobei der Aussenabschnitt der Stromversorgungsleitung (48) ein Ende mit einer Buchse (40) aufweist und der wenigstens eine Innenabschnitt der Stromversorgungsleitung (46, 47) ein Ende mit einem Stecker (41) aufweist, wobei das Ende mit dem Stecker (41) sich aus dem wenigstens einen Arm (10) herauserstreckt, um mit der Buchse (40) des Aussenabschnitts der Stromversorgungsleitung (48) verbunden zu werden.

5. Medizinisches Instrument (1) nach einem der vorangehenden Ansprüche 3-4, **dadurch gekennzeichnet, dass** die wenigstens eine Kavität (100) einen ersten Kavitätsabschnitt (101) aufweist, der in einen longitudinalen Körper (15) des wenigstens einen Arms (10) eingearbeitet ist, und einen zweiten Kavitätsabschnitt (102) aufweist, der in die Wand (30) des wenigstens einen Arms (10) eingearbeitet ist, wobei die Wand (30) mit dem longitudinalen Körper (15) verschiebbar gelagert ist, wobei das wenigstens eine Stromversorgungskabel (45) einen ersten Innenabschnitt der Stromversorgungsleitung (46), der in dem ersten Kavitätsabschnitt (101) eingebaut ist, einen zweiten Innenabschnitt der Stromversorgungsleitung (47), der in dem zweiten Kavitätsabschnitt (102) eingebaut ist, und einen elektrischen Kontaktstellen- bzw. Bürsten (49)-Verbinder zum elektrischen Verbinden des ersten Abschnitts (46) und des zweiten Abschnitts der Stromversorgungsleitung (47) aufweist.

6. Medizinisches Instrument (1) nach einem der vorangehenden Ansprüche 1-5, **dadurch gekennzeichnet, dass** die mehreren Belichtungsquellen (51-53) in einer sterilen Dichtung (55) eingebaut sind.

7. Medizinisches Instrument (1) nach einem der vorangehenden Ansprüche, **dadurch gekennzeichnet, dass** die mehreren Beleuchtungsquellen (51-53) mit einem elektronischen Instrument (6) logisch verknüpft sind, das wenigstens einen Speicher (61) und wenigstens einen Prozessor (62) aufweist, wobei der wenigstens eine Speicher (61) Schritte wenigstens einer ersten Prozedur beinhaltet, die durch den wenigstens einen Prozessor (62) durchgeführt wird, wobei die wenigstens eine erste Prozedur wenigstens einen ersten Zündungsschritt der wenigstens einen ersten Beleuchtungsquelle (51) der Mehrzahl von Beleuchtungsquellen (51-53) für wenigstens eine erste Zeitdauer (T1) und wenigstens einen zweiten Zündungsschritt der wenigstens einen zweiten Beleuchtungsquelle (52) der Mehrzahl von Beleuchtungsquellen (51-53) für wenigstens eine zweite Zeitdauer (T2) beinhaltet, wobei die Zeitdauern (T1, T2) in dem wenigstens einen Speicher (61) festgelegt werden.

8. Medizinisches Instrument (1) nach Anspruch 7, **dadurch gekennzeichnet, dass** der wenigstens eine Prozessor (62) wenigstens den wenigstens einen ersten Zündungsschritt und wenigstens einen von dem wenigstens einen zweiten Zündungsschritt sequentiell oder gleichzeitig ausführt.

9. Medizinisches Instrument (1) nach einem der vorangehenden Ansprüche 7 oder 8, **dadurch gekennzeichnet, dass** der wenigstens eine Speicher (61) Schritte einer zweiten, durch den wenigstens einen Prozessor (62) ausgeführten Prozedur beinhaltet, wobei die zweite Prozedur wenigstens einen ersten Schritt für ein Vergrößern einer Beleuchtungsintensität von wenigstens einer Beleuchtungsquelle (51-53) der Mehrzahl von Beleuchtungsquellen (51-53) sowie wenigstens einen zweiten Schritt für ein Verkleinern der Beleuchtungsintensität der wenigstens einen Beleuchtungsquelle (51-53) beinhaltet.

10. Medizinisches Instrument (1) nach einem der vorangehenden Ansprüche 7-9, **dadurch gekennzeichnet, dass** eine Fernsteuereinrichtung (65) den Prozessor (62) befehligt, in dem wenigstens einen Speicher (61) die Zeitdauern (T1, T2) festlegt, und in dem wenigstens einen Speicher (61) eine Sequenz von Schritten festlegt.

## Revendications

1. Instrument médical (1) comprenant au moins deux bras (10-12), au moins un premier bras (11) et au moins un deuxième bras (12) qui sont adaptés pour passer d'une position de distance mutuelle minimale à une position de distance mutuelle maximale entre eux (11, 12), ladite distance mutuelle étant mesurée selon un axe horizontal (X), chaque bras (10) desdits au moins deux bras (10-12) s'étend longitudinalement le long d'une direction d'un axe longitudinal (Y) étant perpendiculaire par rapport audit axe horizontal (X), chaque bras (10-12) comprend au moins une partie préhensile (3) comprenant une paroi (30) pendante vers le bas le long d'une direction d'un axe vertical (Z), ledit axe vertical (Z) est perpendiculaire à la fois à l'axe horizontal (X) et à l'axe longitudinal (Y), au moins un bras (10) desdits au moins deux bras (10-12) monte un appareil d'éclairage (4) qui comprend une multiplicité de sources lumineuses (51-53) disposées le long de ladite paroi (30), **caractérisé en ce qu'**au moins une première source lumineuse (51) de ladite multiplicité de sources lumineuses (51-53) est disposée à une hauteur qui est plus élevée par rapport à au moins une deuxième source lumineuse (52) de ladite multiplicité de sources lumineuses (51-53), ladite hauteur est mesurable le long de l'axe vertical (Z), ladite multiplicité de sources lumineuses (51-53) émet une multiplicité de faisceaux lumineux dont les intensités maximales sont dirigées le long d'une multiplicité d'axes (H, M, L) divergents les uns des autres et disposés sur un plan géométrique (Σ) identifié par l'axe horizontal (X) et par l'axe vertical (Z).

2. Instrument médical (1) selon la revendication 1, **caractérisé en ce que** ladite au moins une première source lumineuse (51) émet un premier faisceau lumineux dont l'intensité maximale est dirigée le long d'un premier axe divergent (H) de ladite multiplicité d'axes (H, M, L) et ladite au moins une deuxième source lumineuse (52) émet un deuxième faisceau lumineux dont l'intensité maximale est dirigée le long d'un deuxième axe divergent (M) de ladite multiplicité d'axes (H, M, L), ledit premier axe divergent (H) est dirigé vers un premier angle (α) par rapport à ladite paroi (30), ledit premier angle (α) étant situé sur ledit plan géométrique (Σ) et étant compris entre 0 et 90 degrés sexagésimaux et ledit deuxième axe divergent (M) est dirigé vers un deuxième angle (β) par rapport audit premier axe (H), ledit deuxième angle (β) étant situé sur ledit plan géométrique (Σ) et étant compris entre 0 et 270 degrés sexagésimaux.

3. Instrument médical (1) selon l'une quelconque des revendications précédentes 1 ou 2, **caractérisé en ce que** ledit appareil d'éclairage (4) comprend au moins un câble d'alimentation (45) pouvant être monté à l'intérieur d'au moins une cavité (100) dudit au moins un bras (10) dudit instrument médical (1), ladite multiplicité de sources lumineuses (51-53) est connectée électriquement audit au moins un câble d'alimentation (45).

4. Instrument médical (1) selon la revendication 3, **caractérisé en ce que** ledit câble d'alimentation (45) comprend au moins une partie interne d'un câble d'alimentation (46, 47) montée à l'intérieur de ladite au moins une cavité (100) dudit au moins un bras (10) et une partie extérieure d'un câble d'alimentation (48) connectée à une alimentation externe, ladite partie extérieure du câble d'alimentation (48) comprend une extrémité avec une fiche femelle (40) et ladite au moins une partie interne du câble d'alimentation (46, 47) comprend une extrémité avec une fiche mâle (41), ladite extrémité avec une fiche mâle (41) s'étend hors dudit au moins un bras (10) pour être connecté à ladite fiche femelle (40) de ladite partie extérieure du câble d'alimentation (48).

5. Instrument médical (1) selon l'une quelconque des revendications précédentes 3 à 4, **caractérisé en ce que** ladite au moins une cavité (100) comprend une première partie de cavité (101) taillée à l'intérieur d'un corps longitudinal (15) dudit au moins un bras (10) et une deuxième partie de la cavité (102) taillée à l'intérieur de ladite paroi (30) dudit au moins un bras (10), ladite paroi (30) étant montée coulissante avec ledit corps longitudinal (15), ledit au moins un câble d'alimentation (45) comprenant une première partie interne du câble d'alimentation (46) montée à l'intérieur de la première partie de cavité (101), une deuxième partie interne du câble d'alimentation (47) montée à l'intérieur de la deuxième partie de cavité (102) et un connecteur électrique à brosse (49) pour connecter électriquement la première partie (46) et la deuxième partie du câble d'alimentation (47).

6. Instrument médical (1) selon l'une quelconque des revendications précédentes 1 à 5, **caractérisé en ce que** ladite multiplicité de sources lumineuses (51-53) est montée à l'intérieur d'un joint stérile (55).

7. Instrument médical (1) selon l'une quelconque des revendications précédentes, **caractérisé en ce que** ladite multiplicité de sources lumineuses (51-53) est logiquement reliée à un instrument électronique (6) comprenant au moins une mémoire (61) et au moins un processeur (62), ladite au moins une mémoire (61) comprend des étapes d'au moins un première procédé exécuté par ledit au moins un processeur (62), ledit au moins un premier procédé comprenant au moins une première étape d'allumage dudit au moins une première source lumineuse (51) de ladite multiplicité de sources lumineuses (51-53) pendant au moins une première période de temps (T1) et au moins une deuxième étape d'allumage de ladite au moins une deuxième source lumineuse (52) de ladite multiplicité de sources lumineuses (51-53) pendant au moins une deuxième période de temps (T2), lesdites périodes de temps (T1, T2) étant configurées dans ladite au moins une mémoire (61).

8. Instrument médical (1) selon la revendication 7, **caractérisé en ce que** ledit au moins un processeur (62) exécute au moins ladite au moins une première étape d'allumage et au moins une dite au moins une deuxième étape d'allumage séquentiellement ou simultanément.

9. Instrument médical (1) selon l'une quelconque des revendications précédentes 7 ou 8, **caractérisé en ce que** ladite au moins une mémoire (61) comprend des étapes d'un deuxième procédé exécuté par ledit au moins un processeur (62), ledit deuxième procédé comprenant au moins une première étape pour augmenter une intensité lumineuse d'au moins une source lumineuse (51-53) de ladite multiplicité de sources lumineuses (51-53) et au moins une deuxième étape pour diminuer l'intensité lumineuse de ladite au moins une source lumineuse (51-53).

10. Instrument médical (1) selon l'une quelconque des revendications précédentes 7 à 9, **caractérisé en ce qu'**un dispositif de commande à distance (65) commande ledit processeur (62), définit dans ladite au moins une mémoire (61) lesdites périodes de temps (T1, T2), définit dans ladite au moins une mémoire (61) une séquence d'étapes.
